# EUROPEAN PATENT APPLICATION

(11) **EP 4 285 913 A1**
(43) Date of publication of application: **06.12.2023**
(21) Application number: 22176190.1
(22) Date of filing: 30.05.2022
(51) Int. Cl.: A61K 35/17, A61K 38/20, A61K 38/17, A61K 39/00, A61K 39/395, A61P 35/00

(54) **HIGHLY EFFECTIVE ADOPTIVE T CELL THERAPY**

(71) Applicant: Ecole Polytechnique Fédérale de Lausanne (EPFL), 1015 Lausanne (CH)
(72) Inventor: TANG, Li, 1112 Echichens (CH); GUO, Yugang, Hefei, 230022 (CN); FENG, Bing, 1024 Ecublens (CH)
(74) Representative: KATZAROV S.A.

(57) **Abstract**

The present invention relates generally to the field of anti-cancer therapy and autoimmune therapy, in particular to the use of adoptive T cell transfer therapy. More specifically, the present invention relates to compositions, pharmaceutical compositions or methods using IL-4, a fragment or variant thereof, fused to a moiety, to increase the efficacy of an anti-cancer immunotherapy or an autoimmune therapy.

## Description

### FIELD OF THE INVENTION

The present invention relates generally to the field of anti-cancer therapy and autoimmune therapy, in particular to the use of adoptive T cell transfer therapy. More specifically, the present invention relates to compositions, pharmaceutical compositions or methods using IL-4, a fragment or variant thereof, fused to a moiety, to increase the efficacy of an anti-cancer immunotherapy or an autoimmune therapy.

### BACKGROUND OF THE INVENTION

Adoptive T cell transfer therapy, in which autologous tumor-infiltrating lymphocytes (TILs) or bioengineered T cells expanded ex vivo are infused into cancer patients, has proven impressive therapeutic outcomes in patients with certain subtypes of B cell leukaemia, lymphoma, and multiple myeloma in recent years. Nevertheless, the efficacy of ACT in most solid tumors is seriously restricted by numerous biophysical and biochemical barriers. Once entering into tumor tissues and stimulated by persistent antigens, these infused CD8+ T cells will be gradually driven to differentiate into progenitor exhausted CD8+ T cells and terminally exhausted CD8+ T cells. While terminally exhausted CD8+ T cells characterized - by highly tumor-cytolytic capability are crucial to - eliminating solid tumor cells, these subsets are prone to perish due to survival deficit. In contrast to progenitor exhausted T cells, which are responsive to immune checkpoint blockade therapies, terminally exhausted CD8+ T cells with unique transcriptional and epigenetic features fail to respond to most current therapeutics.

Thus, interventions aimed to reinforce the survival and longevity of terminally exhausted CD8+ T cells to boost the ACT efficacy against solid tumors are still urgently needed.

### BRIEF DESCRIPTION OF THE FIGURES

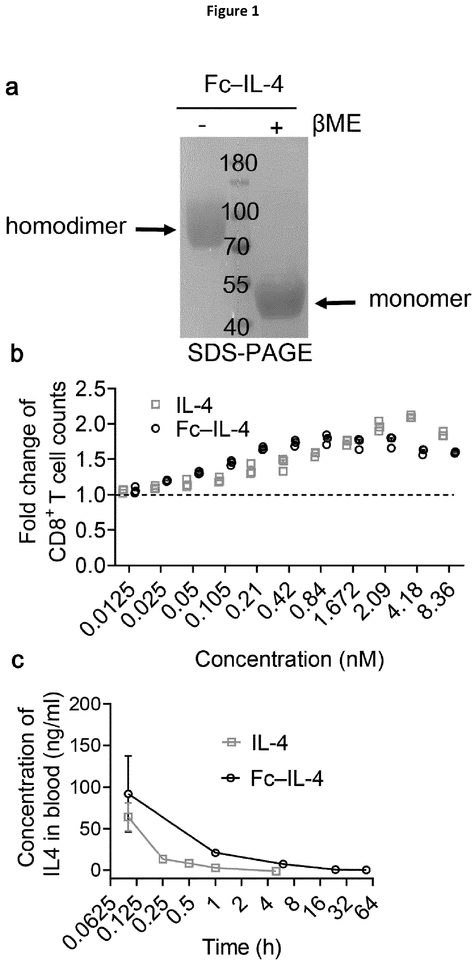

**Fig 1****. Fabrication and characterizations of Fc-IL-4.** a, sodium dodecyl sulfate-polyacrylamide gel electrophoresis (SDS-PAGE) analysis of purified Fc-IL-4. βME, β-mercaptoethanol. b, activated CD8+ T cells were cultured with of IL-4 or Fc-IL-4 at a series of concentrations. Shown is the fold changes of CD8+ T cell counts (normalized by that in PBS control) at each concentration of cytokine. c, IL-4 or Fc-IL-4 were i.v. injected into the C57/BL6J and blood was collected at different time points to measure the concentration of IL-4 and Fc-IL-4 using ELISA kit. Shown is the plasma concentration curve of IL-4 and Fc-IL-4.

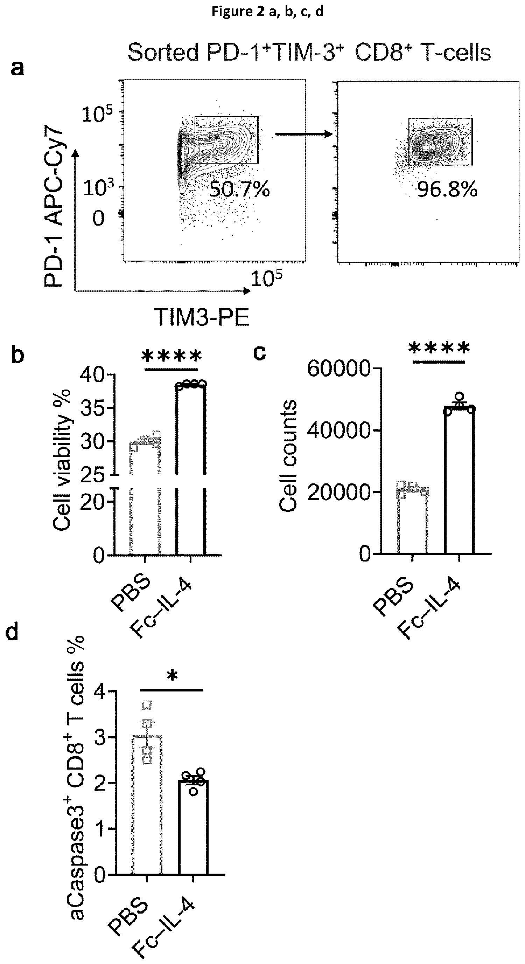

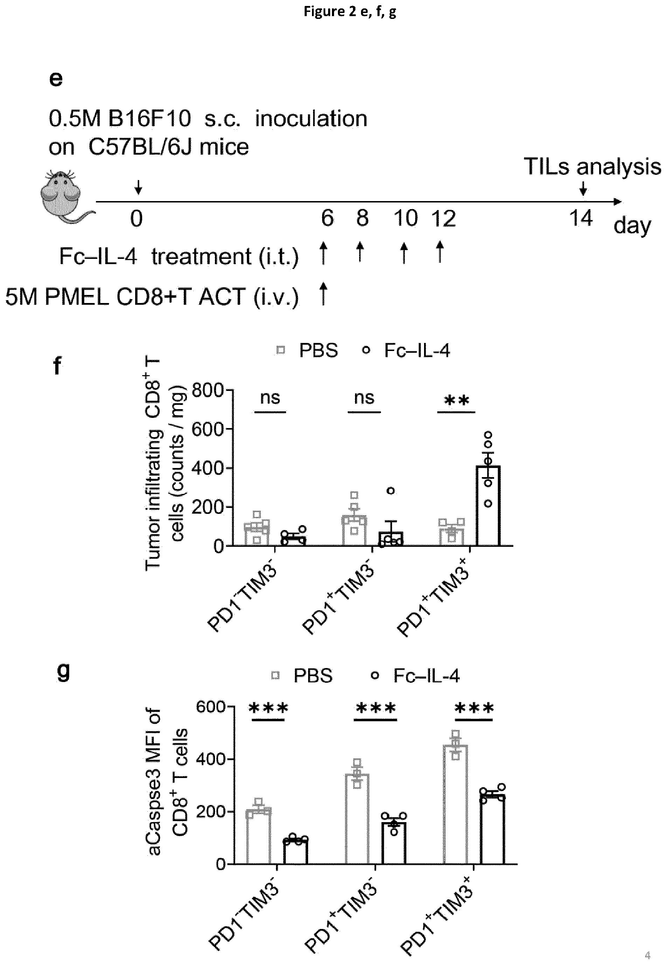

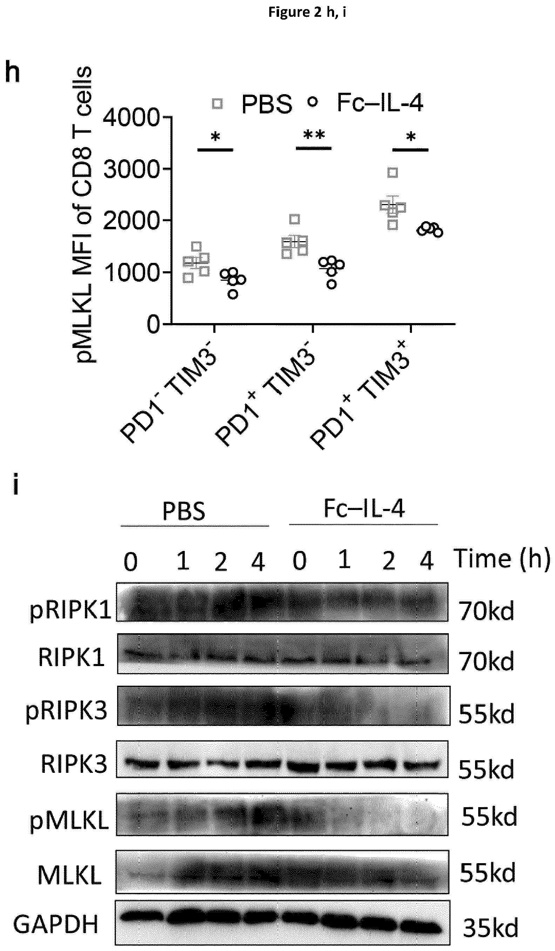

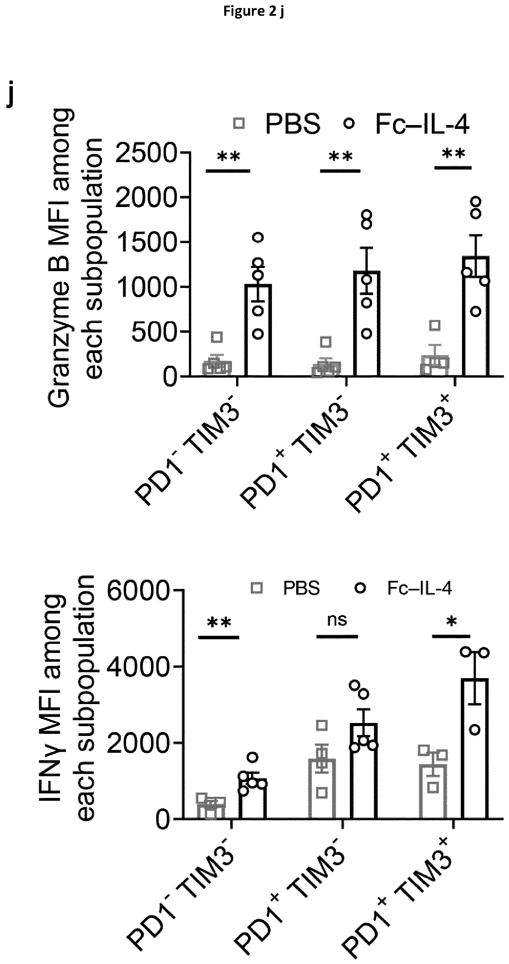

**Fig 2****. Fc-IL-4 improves the survival of terminally exhausted CD8+ T cells population both ex and in vivo.** a, terminally exhausted CD8+ T cells (PD1+TIM3+ CD8+ T cells) were generated ex vivo by re-activation of the resting PMEL-T cells with dimerized CD3 antibodies (α-CD3) for 24 hours and then PD1+TIM3+ CD8+ T cells were sorted using flow cytometry (SONY SH800). Shown is the purified terminally exhausted CD8+ T cells generated ex vivo. b-d, terminally exhausted CD8+ T cells were activated with α-CD3 for 48 hours at the presence of Fc-IL-4 to check the T cell viability (b), cell counts (c) and apoptotic rates (d). e-h, B16F10 tumor-bearing Thy1.2+ C57BL/6 mice received i.v. adoptive cell transfer of activated Thy.1+ PMEL CD8+T cells (5×10⁶ / mouse), and these mice received Fc-IL-4 treatment (i.t., 20 µg / mouse) every other day. One week later after the first Fc-IL-4 treatment, the tumor tissues were collected for tumoral infiltrating lymphocytes analysis. Shown are the experimental timeline (e), tumoral infiltrating CD8+ T cells (f), the active Caspase3 (aCaspase3) mean fluorescence intensity (MFI) value of CD8+ T cells (g), and the phosphorylated MLKL (pMLKL) MFI value of CD8+ T cells (h), respectively. i, ex vivo generated terminally exhausted CD8+ T cells pretreated with Fc-IL-4 for 24 hours were activated with α-CD3 for different time and then collected to measure the activation of necroptois relevant pathways utilizing Western blot (WB). Shown is the WB images and the expression of different necroptois relevant proteins. j, effector function of tumoral infiltrating CD8+ T cells after B16F10 tumor-bearing mice received the treatment as shown in e. Shown is the Granzyme B and IFNγ MFI value of CD8+ T cells.

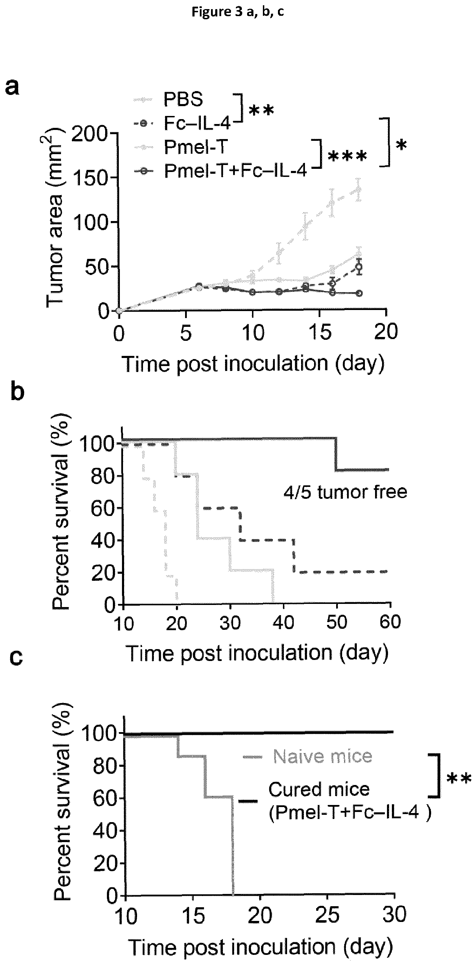

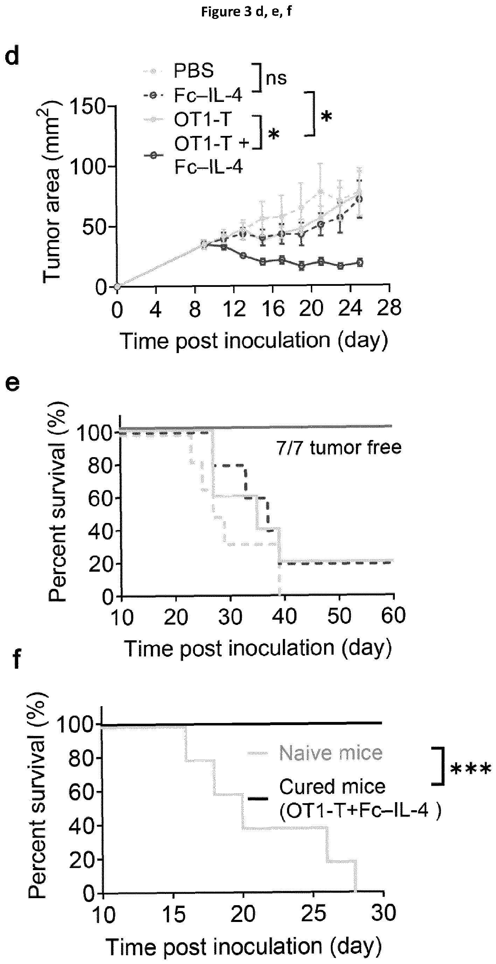

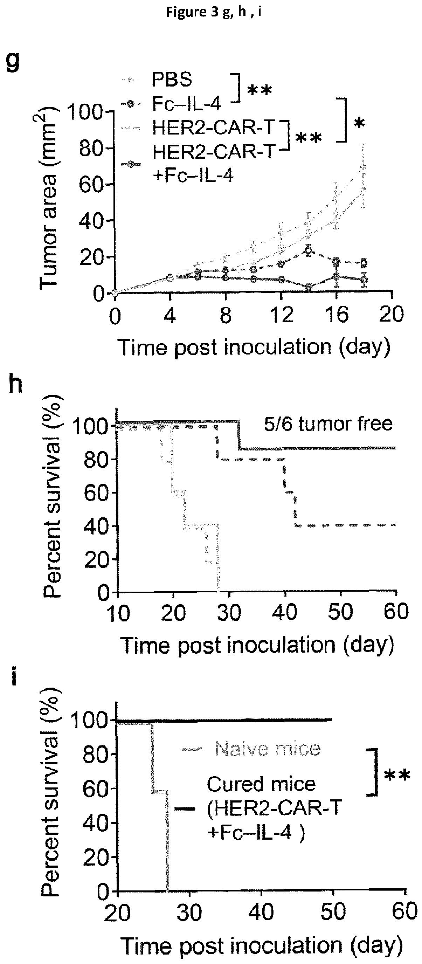

**Fig 3****. Fc-IL-4 potentiates efficacy of ACT in multiple tumor models.** C56BL/6 mice were subcutaneously inoculated with B 16F10 melanoma cells (5×10⁵ / mouse), YUMM1.7-OVA melanoma cells (1×10⁶ / mouse), or MC38-HER2 colon adenocarcinoma cells (5×10⁵ / mouse) and tumor-bearing mice received i.v. adoptive cell transfer of activated PMEL CD8+T cells (5×10⁶ / mouse), OT-1 CD8+ T cells (5×10⁶ / mouse) or HER2-CAR-T cells (5×10⁶ / mouse) and followed by eight i.t. injection of Fc-IL-4 (20 µg / mouse) or PBS control every other day. Mice receiving injections of PBS, Fc-IL-4 (20 µg × 8) only, or ACT together with PBS served as controls. Shown are average tumor growth curves (a, d, g) and survival curves (b, e, h) of each treatment group in different tumor models. Surviving mice from combination treatment of ACT and Fc-IL-4 were rechallenged subcutaneously with B16F10 (1 × 10⁵), YUMM1.7-OVA (5 × 10⁵) or MC38-HER2 (1 × 10⁵) cells, respectively, three months postprimary tumor inoculation. Shown are survival curves (c, f, i) of surviving mice against the re-challenges.

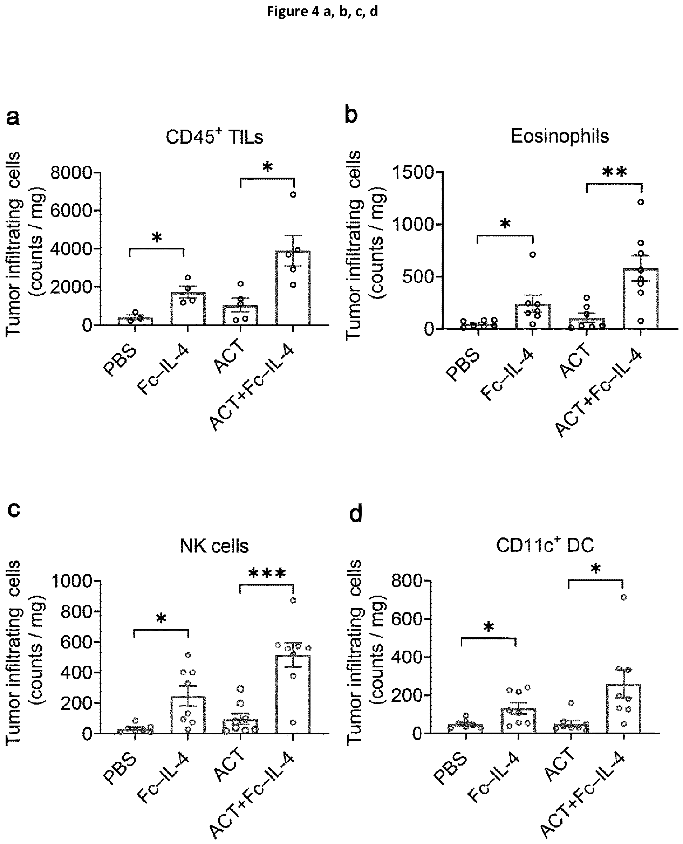

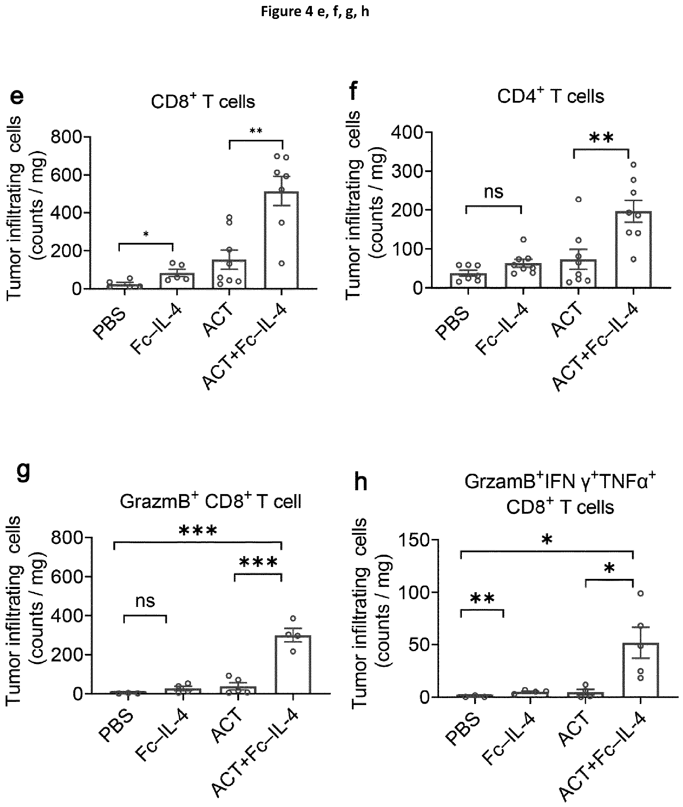

**Fig 4****. Fc-IL-4 significantly improves infiltration of multiple immune cells in the tumor microenvironment.** B16F10 tumor-bearing Thy1.2+ C57BL/6 mice received i.v. adoptive cell transfer of activated Thy.1+ PMEL CD8+T cells (5×10⁶ / mouse), and these mice received Fc-IL-4 treatment (i.t., 20 µg / mouse) every other day. Mice receiving injections of PBS, Fc-IL-4 (20 µg × 4) only, or Thy.1+ PMEL CD8+T cells together with PBS served as controls. One week later after the first Fc-IL-4 treatment, the tumor tissues were collected for tumoral infiltrating lymphocytes analysis. Shown are counts of tumoral infiltrating CD45+ lymphocytes (a), eosinophils (b), NK cells (c), CD11c+ DCs (d), CD8+ T cells (e), CD4+ T cells (f), Granzyme B+ CD8+ T cells (g), and polyfunctional Granzyme B+ IPNγ+TNPα+ CD8+ T cells (h), respectively.

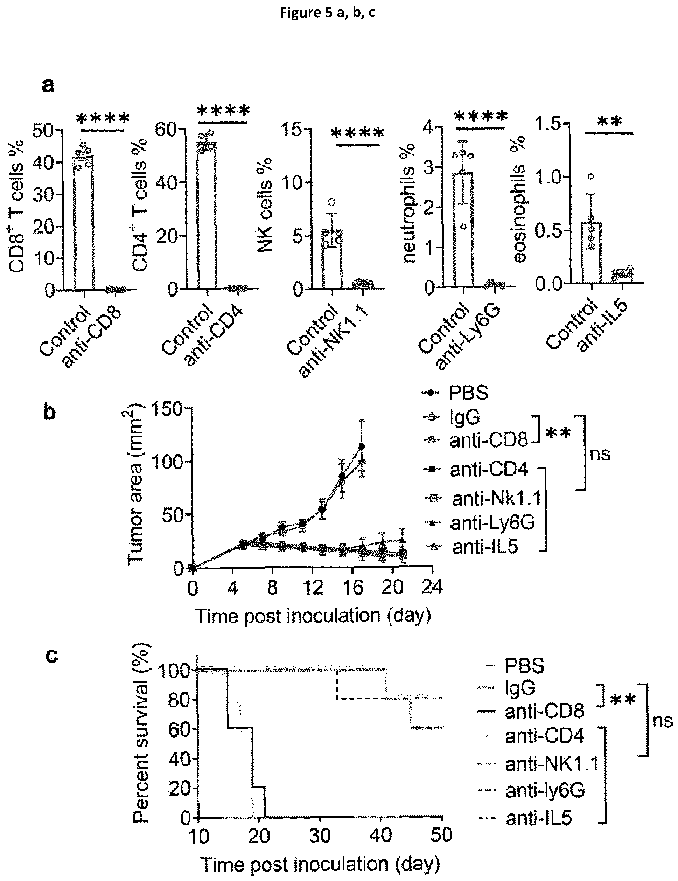

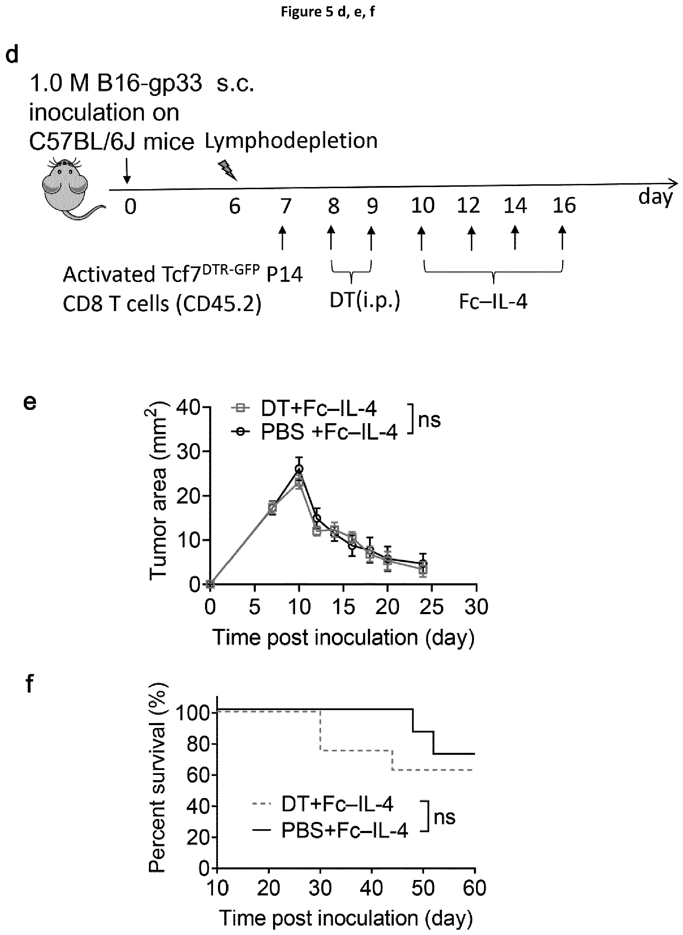

**Fig 5****. CD8+ T cells are the dominant players required for superior efficacy induced by Fc-IL-4.** a-c, B16F10 tumor-bearing Thy1.2+ C57BL/6 mice with area around 25mm² received adoptive cell transfer of activated PMEL CD8+ T cells (5 × 10⁶ / mouse), followed by four i.t. injections of Fc-IL-4 (20 µg / mouse) every other day. Mice were i.p. injected with the anti-CD8 (YTS 169.4, BioXcell, 400 µg / mouse), anti-CD4 (YTS 177, BioXcell, 400 µg / mouse), anti-NK1.1 (PK136, BioXcell, 400 µg / mouse), anti-IL5 (TRFK5, BioXcell, 1 mg / mouse), anti-Ly6G (NIMP-R14 , BioXcell, 400 µg / mouse) or anti-lgG (LTF-2 , BioXcell, 400 µg / mouse), respectively, one day before the treatment and another two injections were given during the treatment to deplete corresponding immune cells and monitor the tumor growth. Shown are the plasma immune cells depletion (a), tumor growth curve (b) and survival curve (c) of each treatment group receiving different antibodies. d-f, B16-gp33 tumor-bearing Thy1.2+ C57BL/6 mice with area around 25mm² received adoptive cell transfer of activated P14+ Tcf7 ^{DTR-GFP} + CD8+ T cells (5 × 10⁶ / mouse) one day after lympho-depletion by whole body irradiation (5 Gy), followed by two i.p. injections of DT (1 µg / mouse) to deplete progenitor exhausted P14 CD8+ T cells and four i.t. injections of Fc-IL-4 (20 µg / mouse) every other day. Shown are the experimental timeline (d), tumor growth curve (e) and survival curve (f) of each treatment group.

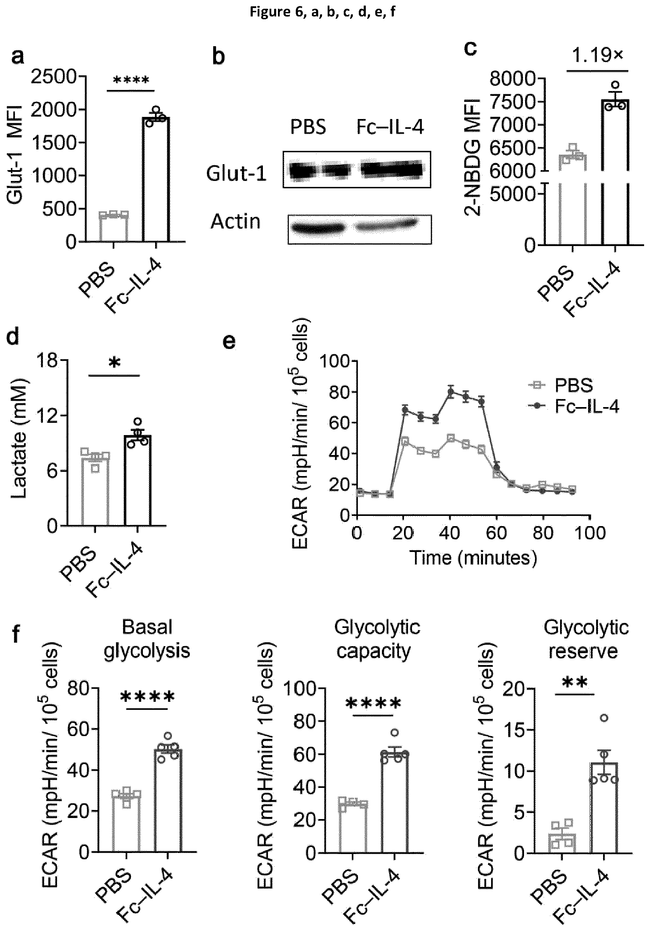

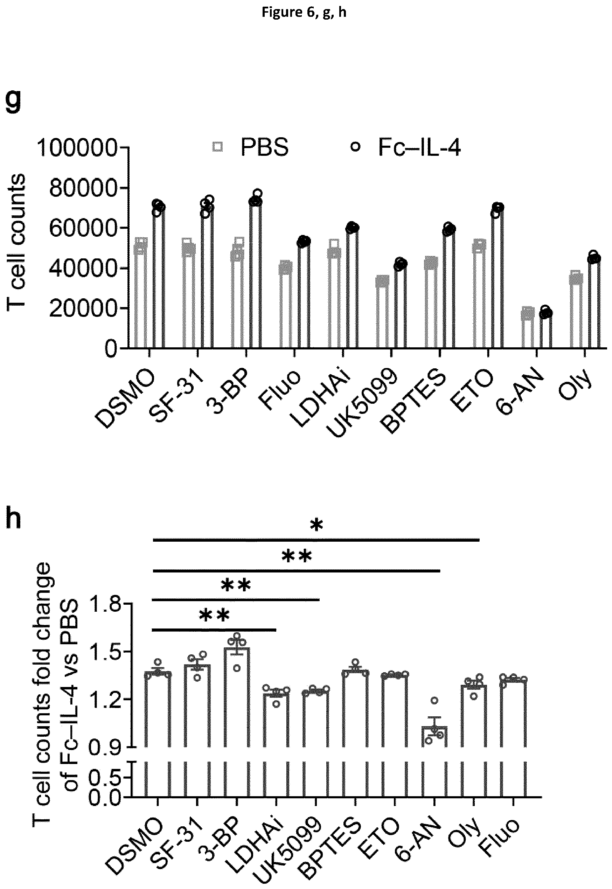

**Fig 6****. Fc-IL-4 reprograms metabolism of CD8+ T cells by enhancing glycolysis.** a - d, ex vivo generated terminally exhausted CD8+ T cells were activated with dimerized α-CD3 (0.5µg / ml) for 48 hours at the presence of Fc-IL-4 (20 ng / ml) to check the metabolic change of CD8+ T cells. Shown are the glucose transporter 1(Glut-1) expression on the cell membrane measured by flow cytometry (a), or western blot (b), the glucose uptake capability (c) and extracellular lactate concentration (d), real-time analysis of ECAR (e), and average basal glycolysis, glycolytic capacity and reserve (f), respectively. g-h, ex vivo generated terminally exhausted CD8+ T cells were activated with dimerized α-CD3 (05µg / ml) for 48 hours at the presence of Fc-IL-4 (20 ng / ml) together with different metabolic inhibitors to assay the cell counts fold change of CD8+ T cells. Shown are the CD8+ T cells counts (g) and fold change (h), after treatment with indicated inhibitors (SF-31, 5 µM; 3-BP 10 µM; Fluoride sodium, ImM; LDHAi, 16 µM; 6-AN, 5 mM; oligomycin, 1 µM; etomoxir (Sigma-Aldrich), 200 µM; BPTES (Sigma-Aldrich), 20 µM; and UK5099 (Sigma-Aldrich), 100 µM).

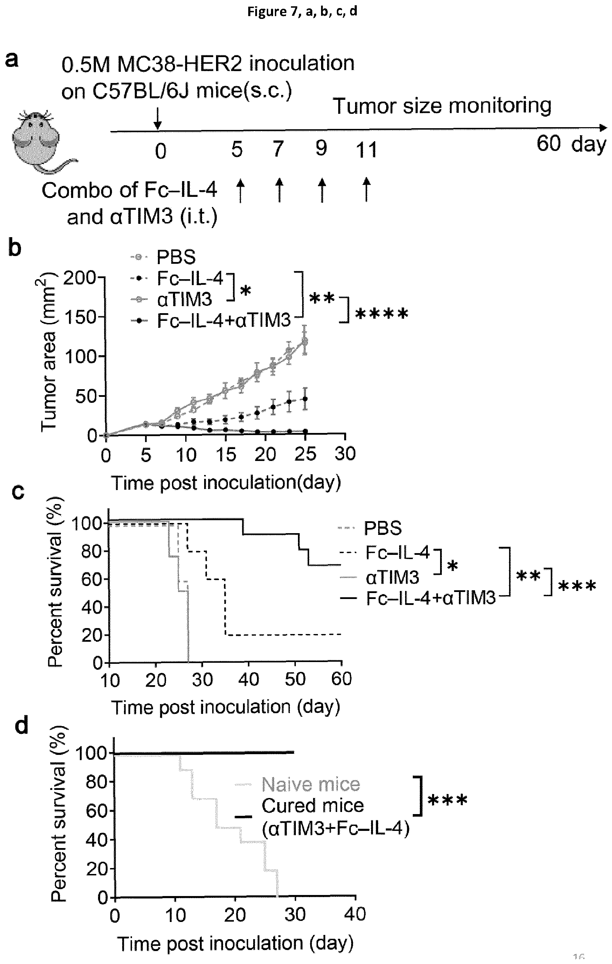

**Fig 7****. Fc-IL-4 synergizes with immune checkpoint blockade and induces durable antitumor effects.** a-c, C56BL/6 mice were subcutaneously inoculated with MC38-HER2 colon adenocarcinoma cells (5×10⁵ / mouse) and tumor-bearing mice received four i.t. injections of Fc-IL-4 (20 µg / mouse) and αTIM3 (50 µg / mouse) every other day. Mice receiving i.t. injections of PBS, Fc-IL-4 (20 µg / mouse × 4) only, or αTIM3 (50 µg / mouse × 4) only served as controls. Shown are the experimental timeline (a), the tumor growth curve (b) and survival curve (c), respectively. Surviving mice from combination treatment of αTIM3 and Fc-IL-4 were rechallenged subcutaneously with MC38-HER2 (1 × 10⁵) cells three months post primary tumor inoculation. Shown are survival curves of surviving mice against the re-challenge (d).

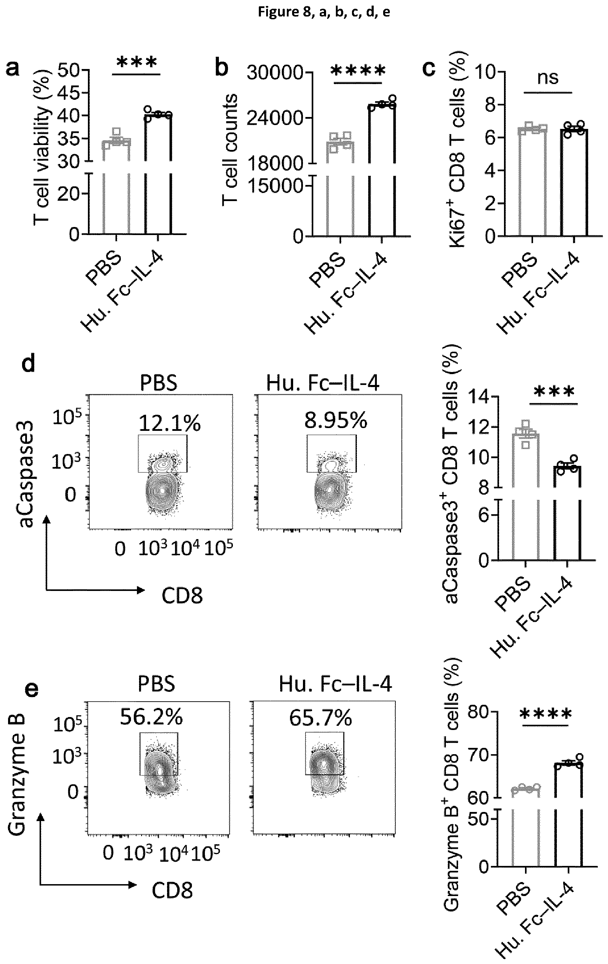

**Fig 8****. Human Fc-IL-4 promotes survival and effector function of human CD8+ T cells.** Human activated CD8+ T cells were activated with human α-CD3 for 48 hours at the presence of hu. Fc-IL-4. Shown are human CD8+ T cell viability (a), cell counts (b), ki67 expression (c), apoptotic rates (d) and granzyme B expression (e), respectively.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise(s)" and "comprising" also encompass the more restricted ones "consist(s)", "consisting" as well as "consist/consisting essentially of", respectively.

As used in the specification and claims, the singular form "a", "an" and "the" include plural references unless the context clearly dictates otherwise.

As used herein, "at least one" means "one or more", "two or more", "three or more", etc. For example, one or more constructs refers to one construct, two constructs, three constructs, etc....

As used herein the terms "subject"/"subject in need thereof", or "patient"/"patient in need thereof" are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human that might be at risk of suffering from a cancer.
According to the present invention, the cancer is a solid or a liquid cancer. In one aspect, the cancer is a solid cancer. Preferably, the solid cancer is selected from the non-limiting group comprising lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer, brain cancer and skin cancer (melanoma), in particular melanoma, or a combination of one or more thereof.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity, i.e., an analogue of A will base-pair with T.

The term "vector", as used herein, refers to a viral vector or to a nucleic acid (DNA or RNA) molecule such as a plasmid or other vehicle, which contains one or more heterologous nucleic acid sequence(s) of the invention and, preferably, is designed for transfer between different host cells and/or for amplification purposes. The terms "expression vector", "gene delivery vector" and "gene therapy vector" refer to any vector that is effective to incorporate and express one or more nucleic acid(s) of the invention, in a cell, preferably under the regulation of a promoter. A cloning or expression vector may comprise additional elements, for example, regulatory and/or post-transcriptional regulatory elements in addition to a promoter.

As used herein, "at least one" means "one or more", "two or more", "three or more", etc.

As used herein, Interleukine-4 (IL-4), refers to a member of cytokine families. IL-4 is a 14 kDa compact globular cytokine, stabilized by three internal disulfide bonds. It was first identified in the early 1980s as a B cell activating factor and exhibits many biological and immunoregulatory functions. As a key regulator in humoral and adaptive immunity, IL-4 acts as a growth and survival factor for lymphocytes, stimulating the proliferation of activated B cells and T cells. In preclinical studies, recombinant, untargeted, murine IL-4 as therapeutic agent showed promising anti-tumor activity in various mouse models of cancer, however, only minimal anti-tumor activity was observed in several clinical studies. Preferably, IL-4 is a human sequence as set forth in SEQ ID NO: 1, a fragment or a variant thereof.
In one aspect the IL-4 is a mouse sequence as set forth in SEQ ID NO: 20, ), a fragment or a variant thereof.

The term "variant", when it refers to IL-4, means one or more biologically active derivatives of an IL-4, preferably of a human IL-4 sequence of the invention. In general, the term "variant" refers to molecules having a native sequence with one or more additions, substitutions (generally conservative in nature) and/or deletions, relative to the native molecule, so long as the modifications do not destroy its biological activity and which are "substantially homologous" to the reference molecule (Gorby et al., Sci. Signal. 13, eabc0653, 2020; Saxton et al., Science 371, eabc8433, 2021). In general, the sequences of such variants will have a high degree of sequence homology or identity to the reference sequence, e.g., sequence homology or identity of more than 25%, generally more than 50% to 70%, even more particularly 80%, or 85% or more, such as at least 90%, or 95% or more, when the two sequences are aligned.

As used herein, a "fragment" of an IL-4, preferably of a human IL-4, of the invention refers to a sequence containing less nucleotides in length than the respective polypeptide sequence or nucleic acid sequence. Preferably, this sequence contains less than 90%, preferably less than 60%, in particular less than 30% nucleotides in length than the respective polypeptide sequence or nucleic acid sequence (SEQ ID No. 1).

While focusing on developing novel and efficient approaches for treating cancer with CAR engineered T cells, the Inventors surprisingly showed that IL-4 fused to a moiety enhancing the half-life of, and/or stabilizing, said IL-4 tremendously increases the antitumor efficacy of anti-cancer immunotherapy such, as ACT, in multiple syngeneic solid tumor bearing mouse models. These results indicate a great potential of IL-4 fused to a moiety to promote the efficacy of an anti-cancer immunotherapy,

The present invention thus provides compositions, as well as pharmaceutical compositions comprising
i) an Interleukin-4 polypeptide (IL-4), a fragment or variant thereof, fused to a moiety, and
ii) an anti-cancer immunotherapy,

comprising a pharmaceutically acceptable carrier, diluent and/or excipient,
wherein the moiety is a molecule enhancing the half-life of, and/or stabilizing, said IL-4, fragment or variant thereof.

Also provided is a pharmaceutical composition comprising
i) an Interleukin-4 polypeptide (IL-4), a fragment or variant thereof, fused to a moiety, and
ii) an anti-cancer immunotherapy,
for use in the treatment and/or prevention of a disease in a subject in need thereof, wherein the moiety is a molecule enhancing the half-life of, and/or stabilizing, said IL-4, fragment or variant thereof.

In an aspect of the invention, the disease is selected from the group comprising a solid cancer, a liquid cancer and an autoimmune disease.

The solid cancer is selected from the non-limiting group comprising lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer and skin cancer, in particular melanoma, or a combination of one or more thereof.

The autoimmune disease is selected from the non-limiting group comprising rheumatoid arthritis (RA), multiple sclerosis (MS), endometriosis, inflammatory bowel disease (IBD), psoriasis, and psoriatic arthritis.

As used herein the terms "subject"/"subject in need thereof", or "patient"/"patient in need thereof" are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human that might be at risk of suffering from a cancer or an autoimmune disease.

In one aspect, the moiety is selected from the group comprising polyethylene glycol (PEG), natural and semi-synthetic polysaccharides, including *O*- and *N-*linked oligosaccharides, dextran, hydroxyethyl starch (HES), polysialic acid and hyaluronic acid, albumin (e.g. Human serum albumin (HSA)), antibody or fragment thereof, protein polymers such as homo-amino acid polymers, elastin-like polypeptides, , XTEN and PAS. In a preferred aspect, the moiety is selected from the group comprising albumin (e.g. HAS as set forth in SEQ ID Nos. 17-19) and an antibody or fragment thereof.

As shown in the exemplary sequences, the moiety can be covalently fused to the IL-4 polypeptide, fragment or variant thereof either at the C-terminal or at the N-terminal end (e.g. moiety-IL-4 or IL-4-moiety). In one aspect, the moiety can be covalently fused to the IL-4 polypeptide, fragment or variant thereof, at the N-terminus or the C-terminus by a polypeptide linker.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, and chimeric antibody, humanized antibody as long as these molecules are still biologically active and still bind to at least one peptide of the invention.

A typical antibody is composed of two immunoglobulin (Ig) heavy chains and two Ig light chains. Several different types of heavy chain exist that define the class or isotype of an antibody. These heavy chain types vary between different animals. All heavy chains contain a series of immunoglobulin domains, usually with one variable (VH) domain that is important for binding antigen and several constant (CH) domains. Each light chain is composed of two tandem immunoglobulin domains: one constant (CL) domain and one variable domain (VL) that is important for antigen binding.

An "antibody fragment", comprises a portion of a full-length antibody. Examples of fragments include Fab, Fc, Fab', F(ab')2, and Fv fragments; diabodies; minobodies; nanobodies; linear antibodies (Zapata et al. (1995) Protein Eng. 8(10):1057-1062); single-chain antibody molecules; and multispecific antibodies formed from antibody fragments. Preferably, the antibody fragment is an Fc domain of an IgG, preferably a silent Fc domain of an immunoglobulin (Ig) G, most preferably of a human IgG 1, IgG2, IgG3 or IgG4. In one aspect, the Fc domain of a human IgG is selected from the group comprising a sequence comprising, or consisting of, IgG1 Fc (SEQ ID No. 2), IgG2 Fc (SEQ ID No. 8), IgG3 Fc (SEQ ID No. 11), and IgG4 Fc (SEQ ID No. 14), a fragment, a variant, or a combination of one or more of these sequences. In one aspect, the Fc domain is a mouse IgG Fc domain selected from the group comprising a sequence comprising, or consisting of, mouse IgG Fc (SEQ ID No. 21), a fragment or a variant thereof.

Usually, the IL-4, fragment, or variant thereof, is covalently fused to the N-terminus or the C-terminus of the Fc domain by a polypeptide linker. In one aspect, the polypeptide linker consists primarily of stretches of Gly and Ser residues ("GS" linker) such as, e.g. as set forth in SEQ ID No. 4 or SEQ ID No. 5.

Any anti-cancer immunotherapy known in the art can be suitable for the present invention. In an aspect of the invention, the anti-cancer immunotherapy is selected from the group comprising Adoptive T cell transfer therapy (ACT), immune checkpoint blockade therapy, cytokine therapy, cancer vaccine therapy, bispecific antibody therapy and other cancer immunotherapies, or a combination of one or more thereof.

ACT is a type of immunotherapy in which T cells are given to a patient to help the body fight diseases, such as cancer. In cancer therapy, T cells are usually taken from the patient's own blood or tumor tissue, grown in large numbers in the laboratory, and then given back to the patient to help the immune system fight the cancer. Sometimes, the T cells are changed in the laboratory to make them better able to target the patient's cancer cells and kill them. Non-limiting types of adoptive cell transfer include T-cell receptor (TCR)-based adoptive therapy (TCR-T), Chimeric antigen receptor T-cell (CAR-T) therapy, Tumor-infiltrating lymphocytes (TILs) and Natural killer (NK) cell therapy, CAR-NK cell, CAR-NKT cell, TCR-transgenic NK cell, TCR-transgenic NK-T cell, CAR-macrophage or any synthetic tumor specific immune cells, or a combination of one or more thereof. ACT is also called adoptive cell therapy, cellular adoptive immunotherapy, and T-cell transfer therapy.

T cell, chimeric antigen receptor (CAR)-T cell, T cell receptor (TCR)-transgenic T cell, tumor infiltrating lymphocyte (TIL), NK cell, NK-T cell, CAR-NK cell, CAR-NKT cell, TCR-transgenic NK cell, TCR-transgenic NK-T cell, dendritic cell, macrophage, CAR-macrophage or any synthetic tumor specific immune cells

Preferably, in Immune checkpoint blockade therapy (ICBT), inhibitors are selected from the non-limiting group a CTLA-4 inhibitor, a TIM3 inhibitor, a TIGIT inhibitor, a PD-1 inhibitor, and a PD-L1 inhibitor or a combination of one or more thereof, e.g. PD-1 / PD-L1 inhibitor or TIM3/PD-1 / PD-L1 inhibitor.

Non-limiting examples of PD-1 inhibitors include nivolumab (Opdivo^{®}), pembrolizumab (Keytruda^{®}), pembrolizumab, pidilizumab, and atezolizumab.

Non-limiting examples of PD-L1 inhibitors include atezolizumab, avelumab, AMP-224, MEDI-0680, RG-7446, GX-P2, durvalumab, KY-1003, KD-033, MSB-0010718C, TSR-042, ALN-PDL, STI-A1014, CX-072, and BMS-936559.

Non-limiting examples of CTLA-4 inhibitors include ipilimumab (Yervoy) (also known as BMS-734016, MDX-010, MDX-101) and tremelimumab (formerly ticilimumab, CP-675,206).

As shown in the examples, the pharmaceutical composition of the invention increases the efficacy of the anti-cancer therapy, in particular the immunotherapy efficacy, by reinforcing the survival and the longevity of the T-cell effectors (and thus their numbers) to boost the immunotherapy efficacy. In one aspect, the pharmaceutical composition of the invention reinforces the survival and longevity of terminally exhausted T-cells and also specifically enriches tumor specific PD-1+TIM-3+CD8+ T cells in the tumor microenvironment by rescuing them from necroptosis and prolonging their survival.

In one aspect, the pharmaceutical composition comprising the IL-4, fragment or variant thereof, fused to a moiety of the invention, increases the efficacy of the anti-cancer immunotherapy of an increase equal or superior to about 2%, equal or superior to about 5 %, equal or superior to about 20 %, equal or superior to about 40 %, equal or superior to about 60 %, equal or superior to about 500%, when compared to the efficacy of the anti-cancer therapy in the absence of the IL-4, fragment or variant thereof, fused to the moiety.

The present invention further contemplates methods of treatment and/or prevention of a disease.

The term "treatment" or "treating" means any administration of a composition, pharmaceutical composition, therapeutic agent, compound, combinaison of compounds, *etc...* of the disclosure to a subject for the purpose of:
(i) inhibiting the disease, that is, arresting the development of clinical symptoms; and/or
(ii) relieving the disease, that is, causing the regression of clinical symptoms.

As used herein, the term "prevention" or "preventing" means any administration of a composition, pharmaceutical composition, therapeutic agent, compound, combinaison of compounds, *etc...* of the disclosure to a subject for the purpose of:
(i) preventing the disease, that is, causing the clinical symptoms of the disease not to develop.

In the context of the present invention, the disease is a cancer or an autoimmune disease.

In one aspect, the invention contemplates a method of treating and/or preventing a cancer or an autoimmune disease, said method comprising administering in a subject in need thereof i) a therapeutically effective amount of at least one IL-4, a fragment or variant thereof, fused to a moiety, wherein the moiety is a molecule enhancing the half-life of, or stabilizing, said IL-4, fragment or variant thereof, and ii) simultaneously or concurrently, a therapeutically effective amount of an anti-cancer therapy or an autoimmune disease therapy.

In one aspect, the method of treatment and/or prevention of a cancer or autoimmune disease in a subject in need thereof comprises (i) removing and isolating immune cells, preferably native T cells, from said patient or subject, (ii) genetically engineering said T cells with one or more recombinant construct encoding a chimeric antigen receptor (CAR), a T cell receptor (TCR) or any other synthetic tumor targeting motif or antigen, (iii) expanding *ex vivo* into a larger population of engineered T cells, and (iv) reintroducing said engineered T cells, into the subject in need thereof, simultaneously or concurrently, with a therapeutically effective amount of at least one IL-4, a fragment or variant thereof, fused to a moiety, wherein the moiety is a molecule enhancing the half-life of, or stabilizing, said IL-4, fragment or variant thereof.

In one aspect the method of treatment and/or prevention of a cancer or an autoimmune disease comprises (i) removing and isolating immune cells, preferably native T cells, from a patient or subject, or providing immune cells, preferably native T cells, (ii) genetically engineering said T cells with at least one recombinant construct encoding a chimeric antigen receptor (CAR), a T cell receptor (TCR) or any other synthetic tumor targeting motif or antigen, (iii) expanding *ex vivo* into a larger population of engineered T cells, and (iv) reintroducing into the patient or subject and (iv) reintroducing said engineered T cells, into the subject in need thereof, simultaneously or concurrently, with a therapeutically effective amount of at least one IL-4, a fragment or variant thereof, fused to a moiety, wherein the moiety is a molecule enhancing the half-life of, or stabilizing, said IL-4, fragment or variant thereof.

In one aspect the method of treatment and/or prevention of a cancer or an autoimmune disease comprises (i) removing and isolating immune cells, preferably native TIL cells, from a patient or subject, or providing immune cells, preferably native TIL cells, (ii) expanding ex *vivo* into a larger population of TIL cells, and (iii) reintroducing said TIL cells, into the subject in need thereof, simultaneously or concurrently, with a therapeutically effective amount of at least one IL-4, a fragment or variant thereof, fused to a moiety, wherein the moiety is a molecule enhancing the half-life of, or stabilizing, said IL-4, fragment or variant thereof.

In one aspect the methods of treatment and/or prevention of a cancer or an autoimmune disease in a subject described above comprise administering a pharmaceutical composition of the invention to a subject in need thereof.

In a one aspect, the methods of treatment and/or prevention described above, can further comprise administrating at least one additional therapeutic agent or therapy, preferably an anticancer agent or anticancer therapy (i.e. when the disease is cancer), more preferably a therapeutically effective amount or dose of an anticancer agent or anticancer therapy. Said one or more anti-cancer agent or therapy will be selected among the non-limiting group comprising radiotherapy, chemotherapy, immune checkpoint inhibitor, immunotherapy and hormone therapy, or a combination of one of more thereof.

The term "therapeutically effective amount" as used herein means an amount of an agent or therapy or combination of compounds, high enough to significantly positively modify the symptoms and/or condition to be treated, but low enough to avoid serious side effects (at a reasonable risk/benefit ratio), within the scope of sound medical judgment.

The present invention further contemplates IL-4 fused to a moiety enhancing the half-life of, or stabilizing, said IL-4 selected from the group comprising, or consisting of, an amino acid sequence as set forth in SEQ ID No. 3, SEQ ID No. 6, SEQ ID No. 7, SEQ ID No. 9, SEQ ID No. 10, SEQ ID No. 12, SEQ ID No. 13, SEQ ID No. 15, SEQ ID No. 16, SEQ ID No. 18, SEQ ID No. 19, and SEQ ID No. 22, a fragment or a variant thereof of anyone of these sequences.

The present invention also contemplates nucleic acid sequences encoding one or more IL-4 fused to a moiety enhancing the half-life of, or stabilizing, said IL-4 described herein. In a preferred aspect, the nucleic acid sequence encodes one or more IL-4 fused to a moiety, as set forth in amino acid sequences selected from SEQ ID Nos 6, 7, 9, 10, 12, 13, 15, 16, 18 and 19.

The present invention further provides a plasmid or a vector comprising a nucleic acid sequence encoding one or more recombinant constructs described herein.

Any vector known in the art can be suitable for the present invention. In some aspects, the vector is a viral vector. In some aspects, the vector is a retroviral vector (such as pMSGV), a DNA vector, a murine leukemia virus vector, an SFG vector, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector (such as pGAR), or any combination thereof.

### EXAMPLES

### Materials and Methods

**Mice.** Six-to-eight-week-old female CD45.2⁺Thy1.2⁺ C57BL/6 (C57BL/6J) mice were purchased from Charles River Laboratories (Lyon, France), TCR-transgenic Thy1.1+ pmel-1 (PMEL) mice (B6.Cg-Thy1a/Cy Tg(TcraTcrb)8Rest/J) and TCR-transgenic OT-I mice (C57BL/6-Tg(TcraTcrb)1100Mjb/J) were originally purchased from the Jackson Laboratory. All mice were maintained in the École Polytechnique Fédérale de Lausanne (EPFL) Center of PhenoGenomics animal facility. Experimental procedures in mouse studies were approved by the Swiss authorities (Canton of Vaud, animal protocol IDs 3206 and 3533) and performed in accordance with the guidelines from the Center of PhenoGenomics of the EPFL.

**Human blood samples.** All human blood samples (prepared as buffy coats) were purchased from Interregional Blood Transfusion SRC Ltd. with informed consent of anonymous healthy donors, and genetically engineered with ethics approval from the Federal Office of Public Health, Switzerland (Notification 4182209/2).

**Cells and tumor models.** B16F10 melanoma cells were originally acquired from the American Type Culture Collection. YUMM1.7-OVA mouse melanoma cell lines and HER2-transduced MC38 mouse colon cancer cell line (MC38-HER2) were generated as previously reported. All the mouse tumor cells were cultured in complete DMEM, a DMEM (Gibco/Thermo Fisher Scientific) supplemented with fetal bovine serum (FBS) (10% v/v, Gibco/Thermo Fisher Scientific) and HEPES (pH 7.2~7.5, 1% v/v, Gibco/Thermo Fisher Scientific) and penicillin/streptomycin (1% v/v, Gibco/Thermo Fisher Scientific). B16F10, YUMM1.7-OVA, or MC38-HER2 tumor cells (5 × 10⁵ or 1 × 10⁶ or as indicated) were implanted subcutaneously into the right flanks of Thy1.2+ C57BL/6 WT mice to establish the syngeneic tumor models.

**Production of mouse Fc-IL-4 and Human Fc-IL-4 protein.** Both of murine Fc-IL-4 and human Fc-IL-4 fusion were expressed by FreeStyle 293-F Cells (Gibco/Thermo Fisher Scientific) at the EPFL Protein Expression Core Facility. Supernatant of culture medium containing recombinant protein was first filtered through a 0.22-µm membrane to obtain a clear solution. The recombinant protein was captured with a HiTrap Protein A affinity chromatography column on an AKTA pure 25 (GE Healthcare), and eluted with an elution buffer (0.05 M sodium citrate, 0.3 M sodium chloride, pH 3.0). The eluted protein was collected immediately in a neutralization buffer (1 M Tris-HCl, pH 10.0), followed by concentration with membrane ultrafiltration (molecular weight cut-off 10 kDa) in a Vivaspin (GE Healthcare). The concentrated protein solution was further purified with a Superdex 200 Increase size-exclusion chromatography column (GE Healthcare). The purified protein was aliquoted and stored at -80 °C before use. The purity of recombinant protein was confirmed with SDS-PAGE.

**Preparation of PMEL, WT OT-I, and human CD8⁺ T cells.** Spleens from PMEL or OT-I mice were mechanically meshed through a 70-µm strainer (Fisher Scientific). Red blood cells (RBCs) were lysed with ACK lysis buffer (2 ml per spleen, Gibco/Thermo Fisher Scientific) for 3 min at room temperature. After washing twice with cold PBS (Gibco/Thermo Fisher Scientific), Splenocytes were then resuspended at a cell density of 2 × 10⁶ per ml in complete RPMI medium ((RPMI-1640 (Gibco), FBS (10% v/v), HEPES (pH 7.2~7.5, 1% v/v), penicillin/streptomycin (1% v/v), sodium pyruvate (1% v/v, Gibco/Thermo Fisher Scientific) and 2-mercaptoethanol (0.1% v/v, Gibco/Thermo Fisher Scientific)) supplemented with mouse IL-2 (10 ng ml⁻¹) and IL-7 (1 ng ml⁻¹, PeproTech), as well as human gp10025-33 or OVA257-264 peptide (1 µM, GenScript) for PMEL or OT-I T cells, respectively. After a 3-d culture, live cells were enriched by density gradient centrifugation against Ficoll-Paque PLUS (GE Healthcare), followed by another 2-d culture at a cell density of 0.5~1.0 × 10⁶ per ml in complete RPMI medium supplemented with mouse IL-2 (10 ng ml-1) and IL-7 (10 ng ml-1) to afford activated CD8+ T cells with purity >95% (flow cytometry analyses). Human peripheral blood mononuclear cells from anonymous healthy donors (prepared as buffy coats) were activated in vitro with coated anti-human CD3 (2µg/ml, OKT3, BioLegend) and CD28 (2µg/ml, CD28.2, BioLegend) antibodies in the presence of human IL-2 (10 ng ml⁻¹) for 2 d. Activated human CD8+ T cells were isolated by Ficoll density gradient separation and magnetic-activated cell sorting (MACS) using a human CD8⁺ T cell isolation kit (Miltenyi Biotec) for in vitro assays.

**Preparation of mouse CAR-T cells.** For mouse CAR-T cell preparation, Phoenix-Eco cells were transfected with HER2 CAR-carrying plasmid and pCL-Eco-packaging plasmid using the calcium phosphate method. Virus-containing supernatant was collected every 24 h post transfection. The splenocytes from WT mice were stimulated by mouse T-activator CD3/CD28 Dynabeads for 1 d, and then transduced with the virus-containing supernatant using spin transduction methods. Briefly, virus-containing supernatant was dispensed in non-tissue-culture-treated six-well plate and centrifuged at 2,000g for 2h at 32°C. Six-well plate was first coated with protamine (10µg/ml, Sigma-Aldrich) overnight at 4°C and then blockade with PBS containing FBS (v/v 1%) for 20min before use. The activated T cells suspended in T cells culture medium were added into the virus-loaded six-well plate immediately after aspirating the supernatant and centrifuge at 2,000g for 0.5h at 32°C. Cells were passaged every 24 h for another 4 d before use. Transduction efficiency was determined by staining surface HER2 CAR using biotinylated human Her2/ErbB2 Protein (Acro Biosystems) and avidin-Alexa Fluor 488 conjugate (Invitrogen/Thermo Fisher Scientific) 48 h post transduction. Untransduced T cells activated by Dynabeads were used as control.

**Analyses of tumor-infiltrating immune cells.** B16F10 tumor-bearing thy 1.2⁺ C57BL/6 mice received i.v. adoptive transfer of PMEL CD8+ T cells (5 × 10⁶), followed by four doses (or as indicated) of peritumoral (p.t.) administration of Fc-IL-4 (20 µg) or PBS control every other day. For BrdU experiments, mice were administered BrdU (1 mg, Sigma-Aldrich) via i.p. injection 1 d before tumor collection. After the above treatment, tumors were collected, weighed, mechanically minced and stirred at 1,000 r.p.m. in RPMI-1640 medium with collagenase Type IV (1 mg ml-1, Gibco/ Thermo Fisher Scientific), dispase II (100 µg ml-1, Sigma-Aldrich), hyalurondase (100 µg ml-1, Sigma-Aldrich) and DNase I (100 µg ml-1, Sigma-Aldrich) at 37 °C for 60 min for digestion. RBC lysis was performed on the digested tumor samples with ACK lysing buffer. Tumor-infiltrating leukocytes were then enriched by density gradient centrifugation against Percoll (GE Healthcare), resuspended in PBS with BSA albumin (0.2%, wt/v, Sigma-Aldrich), stained with indicated antibodies and analyzed with flow cytometry.

**Flow cytometry analyses.** For surface marker staining, cells collected in U-bottom 96-well plates were first blocked with anti-mouse CD16/32 antibody (BioLegend) and incubated with indicated antibodies at 4 °C for 20 min, followed by live/dead staining by 4,6-diamidino-2-phenylindole (DAPI, Sigma-Aldrich) or Zombie Aqua Fixable Dye (BioLegend). Cells were then washed with PBS containing BSA (0.2%, wt/v) and resuspended in the same buffer for flow cytometry analyses. For intracellular staining, cells were first stained for surface markers and Aqua Fixable Dye as described above. Next, cells were fixed and permeabilized with a Cytofix/Cytoperm Fixation/Permeabilization Solution Kit (BD Biosciences) for cytokine staining or a Foxp3/Transcription Factor Staining Buffer Set (eBioscience) for BrdU, active caspase-3 and transcription factors staining per the manufacturer's instructions, followed by incubation with indicated antibodies for intracellular staining. For intracellular cytokine staining, cells were first stimulated by a Cell Stimulation Cocktail (protein transport inhibitors included, Invitrogen/ Thermo Fisher Scientific) at 37 °C for 4-6 h. Cells were then processed similarly for surface marker staining and intracellular staining as described above. Data were collected using an Attune NxT Flow Cytometer with Attune NxT Software v.3 (Invitrogen/Thermal Fischer Scientific). Analyses were performed using FlowJo 10.6.1 (Tree Star). Gate margins were determined by isotype controls and fluorescence-minus-one controls.

**Antibodies and reagents for flow cytometry.** The following antibodies or staining reagents were purchased from BioLegend: CD16/32 (93, 101302), Thy1.1 (OX-7,202529), Thy1.2 (30-H12, 10 5343), CD45.1 (A20, B218971), CD45.2 (104, 109814), CD8α (53-6.7, 100714), CD8β (YTS256.7.7, 126606), CD4 (RM4-5, 100526), NK1.1 (PK136, 108740), F4/80 (BM8,123108), CD3ε (17A2, 100306), CD19 (6D5,115520), CD44 (IM7, 103006), CD11c (N418, 117348), I-A/I-E (MHC-II, M5/114.15.2, 107643), Siglec-F (S17007L, 155508), CD80 (16-10A1, 104734), CD86 (GL-1, 105006), Foxp3 (MF-14, 126406), CD11b (M1/70, 101228), Ki67 (16A8, 652424), BrdU (3D4, 364104), Granzyme B (GB11, 515403), IFN-γ (XMG1.2, 505826), TNF-α (MP6-XT22, 506308), IL-2 (JES6-5H4, 503822), IL-10Rα (1B1.3a, 112705), CD69 (H1.2F3, 104512), Gr-1 (RB6-8C5, 202519), PD-1 (29F.1A12, 135216), TIM-3 (RMT3-23, 119706). TCF-1 (C63D9, 2203S) was obtained from Cell Signaling Technology. Active caspase-3 (C92-605) was obtained from BD Biosciences. Alexa Fluor 488-conjugated goat anti-rabbit secondary antibody (A- 11008), MitoTracker Green FM, MitoTracker Deep Red FM and MitoSOX Red mitochondrial superoxide indicators were obtained from Thermo Fisher Scientific.

**In vitro restimulation of CD8+ T cells.** Activated PMEL CD8⁺ T cells in resting phase (day 7 in culture) were restimulated with dimerized anti-CD3 antibody (prepared by mixing anti-CD3 antibody (17A2, Bio X Cell) with goat anti-rat IgG (Invitrogen/Thermo Fisher Scientific) at the molar ratio of 2:1) in complete RPMI medium containing IL-2 (10 ng ml⁻¹) for 2 d. Collected cells were phenotyped with flow cytometry based on the expression level of surface inhibitory receptors (PD-1 and TIM-3). The live CD8⁺ T cells were analyzed by flow cytometry, or isolated by Ficoll density gradient separation for Seahorse assay. The PD-1⁺TIM-3⁺CD8⁺ T cell subset was sorted for in vitro coculture assay or Seahorse assay.

**In vitro coculture of T cells and tumor cells.** B16F10 tumor cells were cultured in complete DMEM as described above. B16F10 tumor cells (0.16M in 2ml complete DMEM medium) were seeded in each well of 6-well plates at 37 °C overnight. Following aspiration of tumor culture medium, activated PMEL CD8⁺T cells in resting phase were added to the tumor cell culture at a T cell/tumor cell ratio of 1:2. After another 2-d coculture, all cells were collected for flow cytometry analyses. To determine the lysis of target cells, the viability of tumor cells from the coculture was measured with DAPI staining and flow cytometry

**Seahorse assay.** Seahorse assay was performed to measure OCR and ECAR of T cells. Pan CD8+ T cells or sorted subsets of mouse CD8+ T cells (3 × 10⁵ per well) with different treatment conditions were seeded in a Seahorse culture plate (Seahorse Bioscience) in a non-CO2 incubator at 37 °C for 40 min. OCR and ECAR were measured by an XF96 Seahorse Extracellular Flux Analyzer (Seahorse Bioscience) following the manufacturer's instructions. During a Seahorse assay, cells were treated with oligomycin (1 µM, Sigma-Aldrich), carbonyl cyanide-4-(trifluoromethoxy)phenylhydrazone (FCCP, 2 µM, Sigma-Aldrich), rotenone (0.5 µM, Sigma-Aldrich), antimycin A (0.5 µM, Sigma-Aldrich), glucose (10 mM, Sigma-Aldrich) and 2-DG (50 mM, Sigma-Aldrich). Each condition was performed with 3-6 replicates in a single experiment.

**Metabolic inhibitor treatments.** Activated PMEL CD8+ T cells in resting phase (day 7 in culture) as described above were restimulated with dimerized anti-CD3 antibody in complete RPMI medium containing IL-2 (10 ng ml⁻¹) and indicated inhibitors (SF-31, 5µM; 3-BP 10µM; Fluoride sodium, ImM; LDHAi, 16 µM; 6-AN, 5Mm; oligomycin, 1 µM; etomoxir (Sigma-Aldrich), 200 µM; BPTES (Sigma-Aldrich), 20 µM; and UK5099 (Sigma-Aldrich), 100 µM) for 2 d in the presence or absence of Fc-IL-4 (20 ng ml⁻¹). Live CD8+ T cell counts were determined by flow cytometry analyses.

**Anti-tumor therapy and rechallenging experiments.** Mice bearing established tumors with area around 25-60 mm² (day 6 post inoculation or as indicated) were treated with adoptive transfer of activated PMEL T cells, OT-I T cells, or HER2 CAR T cells (5 × 10⁶ or as indicated), followed by p.t. administration of Fc-IL-4 (20 µg) or PBS control every other day (or as indicated) starting from day 6 (four or eight doses in total as indicated). Mice receiving p.t. administration of PBS control only, Fc-IL-4 only or untransduced T cells served as controls. Tumor area and body weight were measured every other day. Tumor area was calculated by the formula Area = Length × Width from caliper measurements of two orthogonal diameters. Mice were euthanized when body weight loss was beyond 15% of predosing weight, or tumor area reached 150 mm² (as a predetermined endpoint), or the animal had become moribund. In tumor cell rechallenging experiments, B16F10 (1 × 10⁵), YUMM1.7-OVA (5 × 10⁵), or MC38-HER2 (5 × 10⁵) cells were re-implanted subcutaneously into the left flanks of survived mice from treatment groups at day 90 post primary inoculation. Age-matched naive WT mice were subcutaneously inoculated with the same number of tumor cells as control. Survival of rechallenged mice was monitored for at least another 60 d.

**Immune cells depletion in vivo study.** Mice bearing established B16F10 tumors with area around 25mm² (day 6 post inoculation) were treated with adoptive transfer of activated PMEL T cells (5 × 10⁶), followed by p.t. administration of Fc-IL-4 (20 µg) or PBS control every other day (or as indicated) starting from day 6 (four or eight doses in total as indicated). Mice were i.p. injected with the anti-CD8 (YTS 169.4, BioXcell, 400µg/mouse), anti-CD4 (YTS 177, BioXcell, 400µg/mouse), anti-NK1.1 (PK136, BioXcell, 400µg/mouse), anti-IL5 (TRFK5, BioXcell, 1mg/mouse), anti-Ly6G (NIMP-R14 , BioXcell, 400µg/mouse) or anti-IgG (LTF-2 , BioXcell, 400ug/mouse), respectively, one day before the treatment and another two injections were given during the treatment to deplete corresponding immune cells. Tumor area and body weight were measured every other day. Tumor area was calculated by the formula Area = Length × Width from caliper measurements of two orthogonal diameters. Survival of mice was monitored for at least 60 d.

**Combination therapy of anti-TIM-3 and Fc-IL-4** . Mice bearing established MC38-HER2 tumors with area around 20mm² (day 6 post inoculation) were treated with i.p. injection of anti-TIM3 (RMT3-23, BioXcell, 100µg/mouse) followed by p.t. administration of Fc-IL-4 (20 µg) or PBS control every other day (or as indicated) starting from day 6 (four or eight doses in total as indicated). Tumor area and body weight were measured every other day. Tumor area was calculated by the formula Area = Length × Width from caliper measurements of two orthogonal diameters. Survival of mice was monitored for at least 60 d.

**Immunoblot analysis.** Cultured PMEL CD8+ T cells (day 7) treated with Fc-IL-4 were collected and were lysed in RIPA lysis buffer (Cell Signaling Technology) with 1× protease inhibitor cocktails (Cell Signaling Technology). Before electrophoresis, total cell lysates were diluted with 4× protein sample loading buffer (Invitrogen/Thermo Fisher Scientific) containing 1× reducing reagents (Invitrogen/Thermo Fisher Scientific) and incubated at 95°C for 10 min. Electrophoresis of proteins from total cell lysates was performed with NuPAGE electrophoresis system (Invitrogen/Thermo Fisher Scientific). Proteins were then transferred to PVDF membrane (Invitrogen/Thermo Fisher Scientific) by Trans-Blot SD semidry transfer cell (Bio-Rad) at 18 V for 60 min. The PVDF membranes were blocked with 5% w/v nonfat dry milk (Sigma-Aldrich) and then incubated with the indicated primary antibodies at 4 °C overnight. The bands were visualized using SignalFire ECL reagent (Cell Signaling Technology) after incubation with horseradish peroxidase (HRP)-conjugated antibodies. The primary antibodies of Glut1 (#73015, Cell Signaling Technology) and the secondary antibodies of HRP-linked anti-rabbit IgG (7074S, 1:3,000) were obtained from Cell Signaling Technology. The HRP-conjugated beta actin monoclonal antibody (BA3R, MA5-15739-HRP, 1:2,000) was obtained from Invitrogen/Thermo Fisher Scientific.

**Statistical analysis.** Statistical analysis was performed using GraphPad Prism 9 (GraphPad Software). Data are presented as mean ± s.e.m. unless otherwise indicated. Comparisons of two groups were performed by using two-tailed unpaired Student's t-test. Survival data were analyzed using the log-rank test. No statistically significant differences were considered when P values were larger than 0.05.

### Results

Given the factor IL-4 acts as a growth and survival factor for lymphocytes, we first tried to study whether IL-4 could sustain the survival of terminally exhausted CD8+ T cells. To extend the half-life of IL-4, a recombinant mouse IL-4 (SEQ ID NO: 20 -mouse IL-4) and mutant lgG1 Fc fusion protein (Fc-IL-4, SEQ ID NO: 22 -mouse Fc-IL-4) which kept comparable bioactivity was designed and obtained (Fig.1a, b). The pharmacokinetic study revealed that Fc-IL-4 has an increased circulating half-life of 6.31 h compared to that of IL-4 at 0.2 h. (Fig. 1c).

Terminally exhausted CD8+ T cells were sorted from a mixture of subsets and re-stimulated with anti-CD3 in the presence or absence of Fc-IL-4 (Fig. 2a). Fc-IL-4 markedly increased both the cell viability and the cell counts of terminally exhausted CD8+ T cells (Fig. 2b, c). The apoptosis rate was significantly reduced (Fig. 2d). Thus, we show that Fc-IL-4 could promote the survival of terminally exhausted CD8+ T cells by reducing the apoptosis. To further confirm the above results in vivo, the activated Thy1.1 PMEL-CD8+T cells were infused into B16F10 melanoma tumor-bearing mice when the tumor size reached around 30 mm², and these mice were received Fc-IL-4 treatment every two days via intra-tumor (i.t.) injection (Fig. 2e). Two days later after the fourth injection of Fc-IL-4, all the mice were sacrificed to collect the tumor tissues for TIL analysis. The total cell counts of CD8+T cells within the tumors were remarkably increased after Fc-IL-4 treatment. Particularly, Fc-IL-4 treatment specifically increased the PD-1+TIM-3+CD8+ T cell counts in the solid tumor (Fig. 2f), which was 7.1 and 1.9-fold higher number compared to mice without Fc-IL-4 treatment in adoptively transferred and endogenous CD8+ T cell, respectively. In contrast, the PD-1-TIM-3-CD8+ T cell counts and PD-1+TIM-3-CD8+ T cell counts remained unchanged after the Fc-IL-4 treatment. Consistent with ex vivo results, apoptotic PD1+TIM3+CD8+T cells was significantly restrained (Fig.2g). Moreover, the phosphorylation of MLKL in PD1+TIM3+ CD8+T cells, which will induce T cells necroptosis, was also inhibited by Fc-IL-4 (Fig.2h). Western blot results further confirmed that Fc-IL-4 could rescue PD1+TIM3+ CD8+T cells from necroptosis by suppressing the activation of necroptosis associated pathway (Fig.2i). In addition, the effector function of CD8+ T cells, especially PD1+TIM3+ CD8 + T cells, was also tremendously enhanced in the regard of Granzyme B and IFNγ expression as shown in Fig 2j.

It was reported that terminally exhausted T cells could robustly kill tumor cells and their enrichment was positively associated with better outcomes of tumor control. We next sought to investigate whether Fc-IL-4 could reinforce the antitumor efficacy of ACT against solid tumors. In established B16F10 mouse melanoma model, treatment with Fc-IL-4 or PMEL-T cells alone modestly suppressed the tumor growth but could not eliminate the tumor burden which gradually increased. Dramatically, combination treatment with Fc-IL-4 and PMEL-T cells could significantly regress the tumor, leading to durable cures in 80% (4/5) of mice bearing B16F10 mouse melanoma (Fig. 3a, b). Moreover, all the surviving mice from the combination treatment were endowed with long-term memory effect to reject a secondary challenge of the same B16F10 tumor cells (0.1 million tumor cells per mouse) 2 months post the treatment (Fig. 3c). In another mouse melanoma model (YUMM1.7-OVA) with high tumor burden (size > 40 mm² or 100 mm³) before the treatment, co-administration of Fc-IL-4 and OT-1 CD8+ T cells displayed roust tumor regression to achieve durable cures in 100% (7/7) of mice bearing melanoma tumors, whereas single therapy of Fc-IL-4 or ACT just only achieved very limited tumor control and cure rates (Fig. 3d, e). Similar as the B16F10 melanoma tumor model, all surviving mice from the combination treatment could reject the secondary YUMM1.7-OVA challenge (Fig. 3f). We next extended this combination therapy to CAR-T cells based ACT treatment in MC38-HER2 tumor model, a murine colon adenocarcinoma expressing human epidermal growth factor receptor 2 (HER2). Despite of quite limited tumor growth suppression achieved by HER2-CAR-T alone, Fc-IL-4 potently improved the therapeutic benefits of HER2-CAR-T with eventually about 83% (5/6) cured rate of tumor-bearing mice in combination therapy (Fig. 3g, h). The antitumor immunity induced by Fc-IL-4 and CAR-T was also durable and persistent as surviving mice received the treatment could reject the secondary MC38-HER2 tumor challenge (Fig. 3i). Overall, Fc-IL-4 could robustly promote tumor regression mediated by ACT and induce durable cures in syngeneic mouse models with pre-established solid tumors.

Next we analyzed the immune cells infiltration in B16F10 tumor model to explore the influences of Fc-IL-4 treatment on the tumor immune microenvironment. Total CD45+ immune cells were significantly increased by Fc-IL-4 including both innate and adaptive immune cells (Fig. 4). Particularly, innate immune cells such as eosinophils, NK cells, DCs and macrophages were increased after Fc-IL-4 treatment alone or in combination with ACT, whereas MDSC was unaffected. Both CD8+ T cells and CD4+ T cells were increased after combination treatment with Fc-IL-4 and ACT. Moreover, Fc-IL-4 remarkably enhance the cytolytic capability and polyfunctions of CD8+ T cells in tumors (Fig. 4g, h). Considering that Fc-IL-4 induced remarkable influences on multiple immune cells and many immune cells were also reported to directly contribute to tumor control, like CD8 T cell, NK cell and eosinophils, we next attempted to figure out the dominant immune cells determining the potent antitumor efficacy of the combination therapy through immune cells depletion study utilizing correspnding antibodies (Fig. 5a). We found that the antitumor efficacies of co-administration of Fc-IL-4 and ACT remained unchanged after depletion of CD4+ T cells, NK cells, neutrophils as well as eosinophils. Nevertheless, the therapeutic benefit achieved by combination therapy was completely abrogated after depletion of CD8+ T cells, indicating that CD8+ T cells displayed an essential role for tumor control in the context of combination therapy with Fc-IL-4 and ACT (Fig. 5b, c). As terminally exhausted rather than other CD8+ T cells were the major subsets of CD8 + T cells enriched by Fc-IL-4 as shown above, it is likely that Fc-IL-4 specifically increased terminally exhausted CD8+ T cells populations and effector function in the tumor to improve the therapeutic benefits of ACT in solid tumors. To further confirm the direct contribution of terminally exhausted CD8+ T cells to tumor control, we transferred Tcf7^{DTR-GFP} transgenic P14 T cells, which could recognize gp33 antigen in mice bearing B 16-gp33 tumors and allow a selective depletion of the progenitor terminally exhausted CD8+ T cells by diphtheria toxin (DT) treatment (Fig. 5d). The tumor suppression effect of the combination with Fc-IL-4 and ACT was not affected after DT-mediated depletion of the progenitor exhausted T cells (Fig. 5e, f), indicating that Fc-IL-4 could directly act on terminally exhausted CD8+ T cells for robust anti-tumor suppression.

Next we tried to uncover the mechanisms underlying how Fc-IL-4 prolonged the survival of terminally exhausted CD8+ T cells. The differentiation and function change of CD8+ T cells are closely coupled with metabolic reprogramming and driven by metabolically restrictive tumor microenvironment once CD8+ T cells infiltrated into tumor tissues. Considering that Fc-IL-4 brought robustly survival benefit on terminally exhausted CD8+ T cells, the metabolic changes on this subsets were investigated after Fc-IL-4 treatment. We first found that Fc-IL-4 could tremendously upregulated the glucose transporter -1 (Glut-1) expression and glucose uptake capability of CD8+ T cells (Fig. 6a-c). Moreover, extracellular lactate concentration of CD8+ T cells was increased after treatment with Fc-IL-4, which indicated that Fc-IL-4 was indeed involved in the metabolic modulation of CD8+ T cells (Fig. 6d). We next detected the metabolic activity by seahorse assay and found that Fc-IL-4 significantly increased the glycolysis level of CD8+ T cells in the context of re-stimulation (Fig. 6e), indicated by basal glycolysis, glycolytic capacity as well as glycolytic reserve (Fig. 6f). However Fc-IL-4 did not affect the OXPHOS of T cells. So, Fc-IL-4 modulated the metabolism of CD8+ T cells mainly through enhancing glycolysis. To probe which kinases or factors in glycolysis pathway were indispensable for Fc-IL-4 to prolong CD8+ T cells survival, the metabolic inhibitors were investigated. We found that FX11, a lactate dehydrogenase A (LDHA) inhibitor could abrogate the effects of Fc-IL-4 to expand terminally exhausted T cells (Fig. 6g, h). Together, Fc-IL-4 might prolong terminally exhausted CD8+ T cells survival by enhancing glycolysis in a LDHA dependent pathway.

In addition to potent therapeutic improvement of ACT in solid tumors, we also discovered that Fc-IL-4 could display synergistic antitumor effects with anti-TIM3 (αTIM3) in MC38-HER-2 tumor model (Fig. 7a). Although αTIM3 alone did not show obvious tumor suppression, co-administration of Fc-IL-4 and αTIM3 robustly induced tumor regression with eventually 67% (6/9) cured rate (Fig. 7b, c). The surviving mice could reject the rechallenge of MC38-HER2 tumor cells, suggesting that Fc-IL-4 could act as an adjuvant for immune checkpoint blockade therapy to induce durable and long-term antitumor immunity (Fig. 7d).

The effects of human Fc-IL-4 (SEQ ID NO: 6 -Fc- IL-4 fusion protein 1) on human T cells were also evaluated and we found that human Fc-IL-4 (hu. Fc-IL-4) could markedly increase both the cell viability and cell counts of human CD8+ T cells by reducing the apoptotic rate (Fig. 8a-c). Moreover, consistent to murine CD8+ T cells, the cytotoxic effector function of human CD8+ T cells was also tremendously increased by hu. Fc-IL-4 (Fig. 8d), which all suggest the promising application of huFc-IL-4 against solid tumors in clinics.

In summary, we discovered that Fc-IL-4 potently induced tumor regression and durable antitumor immunity together with ACT or Immune checkpoint blockade (ICB) in multiple established solid mouse tumor models. Preliminary studies revealed that Fc-IL-4 specifically prolonged the survival of terminally exhausted CD8+ T cells by increasing glycolysis in a LDHA-dependent pathway. These studies demonstrated a novel therapeutic intervention using Fc-IL-4 to promote therapeutic benefits of ACT and ICB to more cancer patients as well as providing evidence for anti-tumor features of Fc-IL-4 through prolonging survival and longevity of terminally exhausted CD8+ T cells.

### SEQUENCES

**SEQ ID NO: 1** - **human IL-4**
**SEQ ID NO: 2 - IgG1 Fc** 
EPKSADKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALGAPIEKTISKA
KGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK
**SEQ ID NO: 3 - IL-4-Fc fusion protein 1** 
HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSSEPKSADKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALGAPIEK
TISKAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
**SEQ ID NO: 4 - GGS linker**
   GGSGGSGGSGGSGGSGGSGGS
**SEQ ID NO: 5 - GGGGS linker**
   GGGGSGGGGSGGGGSGGGGS
**SEQ ID NO: 6 -Fc- IL-4 fusion protein 1** 
EPKSADKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALGAPIEKTISKA
KGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGKGGSGGSGGSGGSGGSG
GSGGSHKCDITLQEIIKTLNSLTEQKTLCTELTVTDIFAASKNTTEKETFCRAATVLRQFYS
HHEKDTRCLGATAQQFHRHKQLIRFLKRLDRNLWGLAGLNSCPVKEANQSTLENFLERLKTI
MREKYSKCSS
**SEQ ID NO: 7 -Fc- IL-4 fusion protein 1** 
EPKSADKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALGAPIEKTISKA
KGQPREPQVYTLPPSREEMTKNQVSLTCLVKGFYPSDIAVEWESNGQPENNYKTTPPVLDSD
 
GSFFLYSKLTVDKSRWQQGNVFSCSVMHEALHNHYTQKSLSLSPGKGGGGSGGGGSGGGGSG
GGGSHKCDITLQEIIKTLNSLTEQKTLCTELTVTDIFAASKNTTEKETFCRAATVLRQFYSH
HEKDTRCLGATAQQFHRHKQLIRFLKRLDRNLWGLAGLNSCPVKEANQSTLENFLERLKTIM
REKYSKCSS
**SEQ ID NO: 8 - IgG2 Fc** 
ERKCCVECPPCPAPPVAGPSVFLFPPKPKDTLMISRTPEVTCVVVDVSHEDPEVQFNWYVDG
VEVHNAKTKPREEQFNSTFRVVSVLTVVHQDWLNGKEYKCKVSNKGLPSSIEKTISKTKGQP
REPQVYTLPPSREEMTKNQVSLTCLVKGFYPSDIAVEWESNGQPENNYKTTPPMLDSDGSFF
LYSKLTVDKSRWQQGNVFSCSVMHEALHNHYTQKSLSLSPGK
**SEQ ID NO: 9 - IL-4-Fc fusion protein 2** 
HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSSERKCCVECPPCPAPPVAGPSVFLFPPKPKDTLMISRTPEVTCVVVDVSHEDPEVQFN
WYVDGVEVHNAKTKPREEQFNSTFRVVSVLTVVHQDWLNGKEYKCKVSNKGLPSSIEKTISK
TKGQPREPQVYTLPPSREEMTKNQVSLTCLVKGFYPSDIAVEWESNGQPENNYKTTPPMLDS
DGSFFLYSKLTVDKSRWQQGNVFSCSVMHEALHNHYTQKSLSLSPGK
**SEQ ID NO: 10 -Fc- IL-4 fusion protein 2** 
ERKCCVECPPCPAPPVAGPSVFLFPPKPKDTLMISRTPEVTCVVVDVSHEDPEVQFNWYVDG
VEVHNAKTKPREEQFNSTFRVVSVLTVVHQDWLNGKEYKCKVSNKGLPSSIEKTISKTKGQP
REPQVYTLPPSREEMTKNQVSLTCLVKGFYPSDIAVEWESNGQPENNYKTTPPMLDSDGSFF
LYSKLTVDKSRWQQGNVFSCSVMHEALHNHYTQKSLSLSPGKGGSGGSGGSGGSGGSGGSGG
SHKCDITLQEIIKTLNSLTEQKTLCTELTVTDIFAASKNTTEKETFCRAATVLRQFYSHHEK
DTRCLGATAQQFHRHKQLIRFLKRLDRNLWGLAGLNSCPVKEANQSTLENFLERLKTIMREK
YSKCSS
**SEQ ID NO: 11 - IgG3 Fc** 
ELKTPLGDTTHTCPRCPEPKSCDTPPPCPRCPEPKSCDTPPPCPRCPEPKSCDTPPPCPRCP
APELLGGPSVFLFPPKPKDTLMISRTPEVTCVVVDVSHEDPEVQFKWYVDGVEVHNAKTKPR
EEQYNSTFRVVSVLTVLHQDWLNGKEYKCKVSNKALPAPIEKTISKTKGQPREPQVYTLPPS
REEMTKNQVSLTCLVKGFYPSDIAVEWESSGQPENNYNTTPPMLDSDGSFFLYSKLTVDKSR
WQQGNIFSCSVMHEALHNRFTQKSLSLSPGK
**SEQ ID NO: 12 - IL-4-Fc fusion protein 3** 
HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSSELKTPLGDTTHTCPRCPEPKSCDTPPPCPRCPEPKSCDTPPPCPRCPEPKSCDTPPP
CPRCPAPELLGGPSVFLFPPKPKDTLMISRTPEVTCVVVDVSHEDPEVQFKWYVDGVEVHNA
KTKPREEQYNSTFRVVSVLTVLHQDWLNGKEYKCKVSNKALPAPIEKTISKTKGQPREPQVY
TLPPSREEMTKNQVSLTCLVKGFYPSDIAVEWESSGQPENNYNTTPPMLDSDGSFFLYSKLT
VDKSRWQQGNIFSCSVMHEALHNRFTQKSLSLSPGK
**SEQ ID NO: 13 -Fc- IL-4 fusion protein 3** 
ELKTPLGDTTHTCPRCPEPKSCDTPPPCPRCPEPKSCDTPPPCPRCPEPKSCDTPPPCPRCP
APELLGGPSVFLFPPKPKDTLMISRTPEVTCVVVDVSHEDPEVQFKWYVDGVEVHNAKTKPR
EEQYNSTFRVVSVLTVLHQDWLNGKEYKCKVSNKALPAPIEKTISKTKGQPREPQVYTLPPS
REEMTKNQVSLTCLVKGFYPSDIAVEWESSGQPENNYNTTPPMLDSDGSFFLYSKLTVDKSR
WQQGNIFSCSVMHEALHNRFTQKSLSLSPGKGGSGGSGGSGGSGGSGGSGGSHKCDITLQEI
IKTLNSLTEQKTLCTELTVTDIFAASKNTTEKETFCRAATVLRQFYSHHEKDTRCLGATAQQ
FHRHKQLIRFLKRLDRNLWGLAGLNSCPVKEANQSTLENFLERLKTIMREKYSKCSS
**SEQ ID NO: 14 - IgG4 Fc** 
ESKYGPPCPPCPAPEFEGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLGK
**SEQ ID NO: 15 - IL-4-Fc fusion protein 4** 
HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSSESKYGPPCPPCPAPEFEGGPSVFLFPPKPKDTLMISRTPEVTCVVVDVSQEDPEVQF
NWYVDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK
**SEQ ID NO: 16 -Fc- IL-4 fusion protein 4** 
ESKYGPPCPPCPAPEFEGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLGKGGSGGSGGSGGSGGSGGSG
GSHKCDITLQEIIKTLNSLTEQKTLCTELTVTDIFAASKNTTEKETFCRAATVLRQFYSHHE
KDTRCLGATAQQFHRHKQLIRFLKRLDRNLWGLAGLNSCPVKEANQSTLENFLERLKTIMRE
KYSKCSS
**SEQ ID NO: 17 - Human serum albumin (HSA)**
**SEQ ID NO: 18 - IL-4-HSA fusion protein** 
HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSSGGSGGSGGSGGSGGSGGSGGSDAHKSEVAHRFKDLGEENFKALVLIAFAQYLQQCPF
 
EDHVKLVNEVTEFAKTCVADESAENCDKSLHTLFGDKLCTVATLRETYGEMADCCAKQEPER
NECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVARLSQR
FPKAEFAEVSKLVTDLTKVHTECCHGDLLECADDRADLAKYICENQDSISSKLKECCEKPLL
EKSHCIAEVENDEMPADLPSLAADFVESKDVCKNYAEAKDVFLGMFLYEYARRHPDYSVVLL
LRLAKTYETTLEKCCAAADPHECYAKVFDEFKPLVEEPQNLIKQNCELFEQLGEYKFQNALL
VRYTKKVPQVSTPTLVEVSRNLGKVGSKCCKHPEAKRMPCAEDYLSVVLNQLCVLHEKTPVS
DRVTKCCTESLVNRRPCFSALEVDETYVPKEFNAETFTFHADICTLSEKERQIKKQTALVEL
VKHKPKATKEQLKAVMDDFAAFVEKCCKADDKETCFAEEGKKLVAASQAALGL
**SEQ ID NO: 19 -HSA-IL-4 fusion protein** 
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELR
DEGKASSAKQRLKCASLQKFGERAFKAWAVARLSQRFPKAEFAEVSKLVTDLTKVHTECCHG
DLLECADDRADLAKYICENQDSISSKLKECCEKPLLEKSHCIAEVENDEMPADLPSLAADFV
ESKDVCKNYAEAKDVFLGMFLYEYARRHPDYSVVLLLRLAKTYETTLEKCCAAADPHECYAK
VFDEFKPLVEEPQNLIKQNCELFEQLGEYKFQNALLVRYTKKVPQVSTPTLVEVSRNLGKVG
SKCCKHPEAKRMPCAEDYLSVVLNQLCVLHEKTPVSDRVTKCCTESLVNRRPCFSALEVDET
YVPKEFNAETFTFHADICTLSEKERQIKKQTALVELVKHKPKATKEQLKAVMDDFAAFVEKC
CKADDKETCFAEEGKKLVAASQAALGLGGSGGSGGSGGSGGSGGSHKCDITLQEIIKTL
NSLTEQKTLCTELTVTDIFAASKNTTEKETFCRAATVLRQFYSHHEKDTRCLGATAQQFHRH
KQLIRFLKRLDRNLWGLAGLNSCPVKEANQSTLENFLERLKTIMREKYSKCSS
**SEQ ID NO: 20 -mouse IL-4** 
HIHGCDKNHLREIIGILNEVTGEGTPCTEMDVPNVLTATKNTTESELVCRASKVLRIFYLKH
GKTPCLKKNSSVLMELQRLFRAFRCLDSSISCTMNESKSTSLKDFLESLKSIMQMDYS
**SEQ ID NO: 21 -mouse Fc** 
EPRVPITQNPCPPLKECPPCAAPDLLGGPSVFIFPPKIKDVLMISLSPMVTCVVVAVSEDDP
DVQISWFVNNVEVHTAQTQTHREDYNSTLRVVSALPIQHQDWMSGKEFKCKVNNRALPSPIE
KTISKPRGPVRAPQVYVLPPPAEEMTKKEFSLTCMITGFLPAEIAVDWTSNGRTEQNYKNTA
TVLDSDGSYFMYSKLRVQKSTWERGSLFACSVVHEGLHNHLTTKTISRSLGK
**SEQ ID NO: 22 -mouse Fc-IL-4**

EPRVPITQNPCPPLKECPPCAAPDLLGGPSVFIFPPKIKDVLMISLSPMVTCVVVAVSEDDP
DVQISWFVNNVEVHTAQTQTHREDYNSTLRVVSALPIQHQDWMSGKEFKCKVNNRALPSPIE
KTISKPRGPVRAPQVYVLPPPAEEMTKKEFSLTCMITGFLPAEIAVDWTSNGRTEQNYKNTA
TVLDSDGSYFMYSKLRVQKSTWERGSLFACSVVHEGLHNHLTTKTISRSLGKGGSGGSGGSG
GSGGSGGSGGSHIHGCDKNHLREIIGILNEVTGEGTPCTEMDVPNVLTATKNTTESELVCRA
SKVLRIFYLKHGKTPCLKKNSSVLMELQRLFRAFRCLDSSISCTMNESKSTSLKDFLESLKS
IMQMDYS

### REFERENCES

1. Walz, M., Overbergh, L., Mathieu, C., Kolb, H. & Martin, S. A murine interleukin-4-Ig fusion protein regulates the expression of Th1- and Th2-specific cytokines in the pancreas of NOD mice. Horm. Metab. Res. 34, (2002).
2. Vella, A., Teague, T. K., Ihle, J., Kappler, J. & Marrack, P. Interleukin 4 (IL-4) or IL-7 prevents the death of resting T cells: Stat6 is probably not required for the effect of IL-4. J. Exp. Med. 186, 325-330 (1997).
3. Dufort, F. J. et al. Cutting Edge: IL-4-Mediated Protection of Primary B Lymphocytes from Apoptosis via Stat6-Dependent Regulation of Glycolytic Metabolism. J. Immunol. 179, (2007).

## Claims

1. A pharmaceutical composition comprising
i) an Interleukin-4 polypeptide (IL-4), a fragment or variant thereof, fused to a moiety, and
ii) an anti-cancer immunotherapy,
for use in the treatment and/or prevention of a disease, wherein the moiety is a molecule enhancing the half-life of, and/or stabilizing, said IL-4, fragment or variant thereof.

2. The pharmaceutical composition for use of claim 1, wherein the moiety is selected from the group comprising polyethylene glycol (PEG), natural and semi-synthetic polysaccharides, including *O*- and *N*-linked oligosaccharides, dextran, hydroxyethyl starch (HES), polysialic acid and hyaluronic acid, albumin, antibody or fragment thereof, protein polymers such as homo-amino acid polymers, elastin-like polypeptides, XTEN and PAS.

3. The pharmaceutical composition for use of claim 1 or 2, wherein the antibody fragment is a constant fragment (Fc) domain of an antibody, preferably a silent Fc domain of an immunoglobulin (Ig) G.

4. The pharmaceutical composition for use according to any one of the preceding claims, wherein the disease is selected from the group comprising a solid cancer, a liquid cancer and an autoimmune disease.

5. The pharmaceutical composition for use according to claim 4, wherein said solid cancer is selected from the group comprising lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer and skin cancer, in particular melanoma, or a combination of one or more thereof.

6. The pharmaceutical composition for use according to claim 3, wherein Interleukin-4 polypeptide (IL-4), a fragment or variant thereof, covalently fused to the N-terminus or the C-terminus of the Fc domain by a polypeptide linker.

7. The pharmaceutical composition for use according to any one of the preceding claims, wherein said anti-cancer immunotherapy is selected from the group comprising ACT therapy, immune checkpoint blockade therapy, cytokine therapy, cancer vaccine therapy, bispecific antibody therapy and other cancer immunotherapies, or a combination of one or more thereof.

8. The pharmaceutical composition for use according to claim 7, wherein ACT therapy is selected from the group comprising TCR-T, CAR-T, TILs and NK cell therapy, or a combination of one or more thereof.

9. The pharmaceutical composition for use according to claim 8, wherein immune checkpoint blockade therapy comprises inhibitors selected from the group a CTLA-4 inhibitor, a TIM3 inhibitor, a PD-1 inhibitor, and a PD-L1 inhibitor or a combination of one or more thereof.

10. The pharmaceutical composition for use according to any one of the preceding claims, wherein the sequence of IL-4 is a human sequence as set forth in **SEQ ID NO: 1,** fragment or variant thereof.

11. The pharmaceutical composition for use according to any one of the preceding claims, further comprising a pharmaceutically acceptable carrier, diluent and/or excipient.

12. The pharmaceutical composition for use according to claim 7, wherein said immune checkpoint blockade therapy comprises inhibitors selected from the group comprising a CTLA-4 inhibitor, a TIM3 inhibitor, a PD-1 inhibitor, and a PD-L1 inhibitor, or a combination of one or more thereof.

13. The pharmaceutical composition for use according to any one of the preceding claims, wherein the IL-4, a fragment or variant thereof, fused to a moiety, increases the efficacy of the anti-cancer immunotherapy of an increase equal or superior to about 2%, equal or superior to about 5 %, equal or superior to about 20 %, equal or superior to about 40 %, equal or superior to about 60 %, equal or superior to about 500%, when compared to the efficacy of the anti-cancer therapy in the absence of the IL-4, a fragment or variant thereof, fused to a moiety.

14. The pharmaceutical composition for use according to any one of the preceding claims, wherein the agent useful in cancer therapy or in autoimmune disease therapy is administered simultaneously or concurrently, to the IL-4, fragment or variant thereof, fused to a moiety.

15. A pharmaceutical composition comprising
i) an Interleukin-4 polypeptide (IL-4), a fragment or variant thereof, fused to a moiety, and
ii) an anti-cancer immunotherapy,
comprising a pharmaceutically acceptable carrier, diluent and/or excipient,
wherein the moiety is a molecule enhancing the half-life of, and/or stabilizing, said IL-4, fragment or variant thereof.
